# FASCICULE DE BREVET EUROPEEN

(11) **EP 2 205 172 B1**
(45) Date de publication et mention de la délivrance du brevet: **08.02.2012**
(21) Numéro de dépôt: 08866623.5
(22) Date de dépôt: 16.09.2008
(51) Int. Cl.: A61B 17/70

(54) **DISPOSITIF D'ANCRAGE VERTÉBRAL**
WIRBELVERANKERUNGSVORRICHTUNG
VERTEBRAL ANCHORING DEVICE

(30) Priorité: 17.09.2007 FR 0706501; 21.09.2007 US 974085 P
(43) Date de publication de la demande: 14.07.2010
(73) Titulaire: Clariance, 62000 Dainville (FR)
(72) Inventeur: TORNIER, Alain, F-38330 Saint Ismier (FR)
(74) Mandataire: Schmitt, John
(86) Numéro de dépôt international: PCT/FR2008/001293
(87) Numéro de publication internationale: WO 2009/083659

(56) Documents cités:
- EP-A- 1 570 796
- WO-A-2007/047711
- US-A1- 2005 192 571
- US-A1- 2007 118 123

## Description

La présente invention est relative à un dispositif d'ancrage vertébral comportant des connecteurs d'ancrage osseux venant par l'intermédiaire de vis d'ancrage se fixer aux différents corps osseux ou vertèbres d'une colonne vertébrale.

On connaît d'après le brevet américain US 2 346 346, un dispositif d'ancrage osseux externe comportant des connecteurs reliés entre eux par une tige de liaison, tandis que chaque connecteur est fixé à un support ou au corps osseux par l'intermédiaire d'une vis d'ancrage à tête sphérique.

Chaque connecteur est constitué d'un manchon cylindrique comportant à chaque extrémité un alésage de diamètre différent permettant respectivement la mise en place de la vis au travers dudit manchon et la retenue de la tête sphérique à l'intérieur de ce dernier.

Chaque connecteur comporte un dispositif de blocage permettant simultanément l'immobilisation de la tige de liaison à l'intérieur de manchon et la fixation de ce dernier autour de la tête sphérique de la vis.

On note que pour la mise en place de ce dispositif d'ancrage, il est impératif de lier la vis d'ancrage et le connecteur ensemble avant la fixation de ladite vis avec soit un support soit l'os correspondant.

Dans ce cas, la tête sphérique de la vis se trouve logée à l'intérieur du connecteur entraînant une utilisation difficile de cette dernière pour son ancrage avec le support ou l'os.

On connaît d'après le brevet international WO 2004/047657 un dispositif d'ancrage vertébral comprenant un connecteur, une tige de liaison et une vis d'ancrage poly axiale. La vis poly axiale comportant une tête sphérique et un corps fileté dont le diamètre externe d au sommet des dents du filetage est supérieur à celui externe a de la tête sphérique.

Chaque connecteur du dispositif d'ancrage vertébral est constitué d'un élément de connexion comportant des branches verticales délimitant une ouverture en forme de U, et d'un clip de verrouillage muni d'une vis de pression pour le blocage dans le fond du U de la tige de liaison.

L'élément de connexion de chaque connecteur est percé en son milieu d'un alésage vertical permettant de recevoir à l'opposé de l'ouverture un dispositif de blocage formé d'une bague et d'une douille filetée pour la mise en place et le positionnement dudit connecteur sur la tête sphérique de la vis d'ancrage.

On connait également d'après le brevet international WO 2007/047711 un dispositif d'ancrage selon le préambule de la revendication 1, permettant de fixer un ou plusieurs implants le long de la colonne vertébrale. Chaque dispositif d'ancrage est constitué d'un connecteur recevant d'une part une tige de liaison et des moyens de blocage de cette dernière et d'autre part un élément de liaison en forme de bague fendue permettant la réception et le blocage de la tête à profil sphérique d'une vis d'ancrage.

On connait d'après le brevet US 2007/0118123 un dispositif d'ancrage constitué de connecteurs permettant d'une part la réception d'une tige de liaison et des moyens de blocage en rotation et translation de ladite tige et d'autre par la mise en place d'un bague fendue permettant la retenue de la tête sphérique d'une vis d'ancrage.

L'objet de la présente invention consiste à prévoir un dispositif d'ancrage vertébral dont chaque connecteur comporte des moyens de liaison assurant d'une part le réglage angulaire dudit connecteur autour de la vis d'ancrage et d'autre part l'entraînement en rotation de la vis d'ancrage pour sa fixation dans le corps de la vertèbre.

Le dispositif d'ancrage vertébral suivant la présente invention comprend un dispositif d'ancrage vertébral comprenant des connecteurs reliés entre eux par des tiges de liaison et fixés sur des vis d'ancrage osseux implantés dans les vertèbres d'une colonne vertébrale, chaque connecteur étant constitué d'un élément de connexion comportant d'une part des moyens de réception et de fixation permettant l'immobilisation en translation et en rotation de la tige de liaison et d'autre part à l'opposé des moyens de réception et de fixation de la tige de liaison, un logement coopérant avec un dispositif de liaison constitué d'une douille permettant l'assemblage de la vis d'ancrage, ladite douille assurant avant l'immobilisation de la tige de liaison, dans l'élément de connexion,
- d'une part des basculements latéraux et indépendants de l'élément de connexion et de la vis d'ancrage l'un par rapport à l'autre par l'intermédiaire de premiers moyens de rotation constitués de premiers alésages ménagés respectivement dans le logement de l'élément de connexion et la douille du dispositif de liaison permettant à ladite douille de pouvoir basculer latéralement autour d'axes de rotation coopérant avec lesdits premiers alésage et de seconds moyens de rotation constitués de second alésages ménagés respectivement dans la douille est la tête de la vis d'ancrage permettant à cette dernière de pouvoir basculer latéralement autour d'un autre axe de rotation,
- Et d'autre part l'entraînement en rotation de la vis d'ancrage pour sa fixation dans le corps de la vertèbre

Le dispositif d'ancrage vertébral suivant la présente invention comprend une douille percée en son milieu et suivant une direction verticale d'un alésage débouchant destiné à recevoir la tête de la vis d'ancrage.

Le dispositif d'ancrage vertébral suivant la présente invention comprend une douille percée suivant une première direction horizontale d'un premier alésage débouchant à l'intérieur de l'alésage interne et permettant la mise en place des axes de rotation afin que ces derniers débouchent dans les alésages aménagés dans le logement de l'élément de connexion afin d'assurer le premier basculement latéral.

Le dispositif d'ancrage vertébral suivant la présente invention comprend une douille percée suivant une seconde direction horizontale et perpendiculaire à celle du premier alésage d'un second alésage débouchant également à l'intérieur de l'alésage interne et permettant la mise en place d'un axe de fixation traversant un alésage ménagé dans la tête de la vis d'ancrage afin d'assurer le second basculement latéral.

Le dispositif d'ancrage vertébral suivant la présente invention comprend une douille qui comporte un bord périphérique supérieur présentant d'une part au-dessus de chaque premier alésage débouchant un bossage et d'autre part au-dessus de chaque second alésage et à l'intérieur de l'alésage interne des nervures.

Le dispositif d'ancrage vertébral suivant la présente invention comprend une douille reliée à une bague comportant en son milieu et suivant une direction verticale un trou et sur son pourtour externe des encoches coopérant avec les nervures de ladite douille.

Le dispositif d'ancrage vertébral suivant la présente invention comprend un élément de connexion comportant des moyens de réception et de fixation de la tige de liaison qui sont constitués par des branches verticales supérieures délimitant une ouverture en forme de U pour la réception de ladite tige de liaison, lesdites branches verticales supérieures comprenant au-dessus du fond de l'ouverture une section filetée permettant le serrage d'une vis de pression pour le blocage en translation et en rotation de la tige de liaison.

Le dispositif d'ancrage vertébral suivant la présente invention comporte un élément de connexion comprenant en dessous de la section filetée et à la base de chaque branche verticale une ouverture débouchant à l'intérieur dudit élément de connexion au niveau de la communication entre le fond de l'ouverture et le logement suivant une direction horizontale et perpendiculaire à celle de la tige de liaison.

Le dispositif d'ancrage vertébral suivant la présente invention montre que chaque ouverture de l'élément de connexion disposée l'une en face de l'autre, présente un profil vertical oblong.

Le dispositif d'ancrage vertébral suivant la présente invention montre que les alésages débouchants de l'élément de connexion sont disposés suivant une direction qui est horizontale et perpendiculaire à celle des ouvertures oblongues. Le dispositif d'ancrage vertébral suivant la présente invention comprend d'autres caractéristiques essentielles pour la réalisation de l'invention qui sont décrites et protégées dans les revendications secondaires dépendantes directement ou indirectement de la revendication principale.

Les dessins annexés, donnés à titre d'exemple, permettront de mieux comprendre l'invention, les caractéristiques qu'elle présente et les avantages qu'elle est susceptible de procurer:
Figure 1 est une vue de dessus représentant des corps vertébraux d'une colonne vertébrale dans lesquels sont fixés des connecteurs d'ancrage osseux suivant la présente invention.
Figure 2 est une vue en perspective éclatée illustrant le connecteur d'ancrage osseux et sa vis d'ancrage suivant la présente invention.
Figures 3 à 7 sont des vues montrant l'assemblage du connecteur d'ancrage osseux avec sa vis d'ancrage osseux suivant la présente invention.
Figures 8 et 10 sont des vues représentant le connecteur d'ancrage osseux recevant une tige de liaison suivant la présente invention.

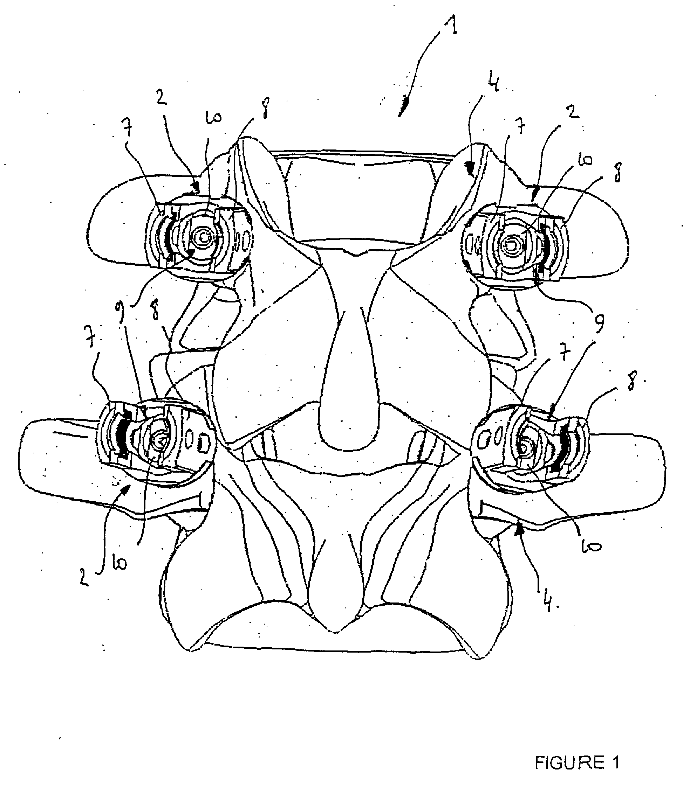

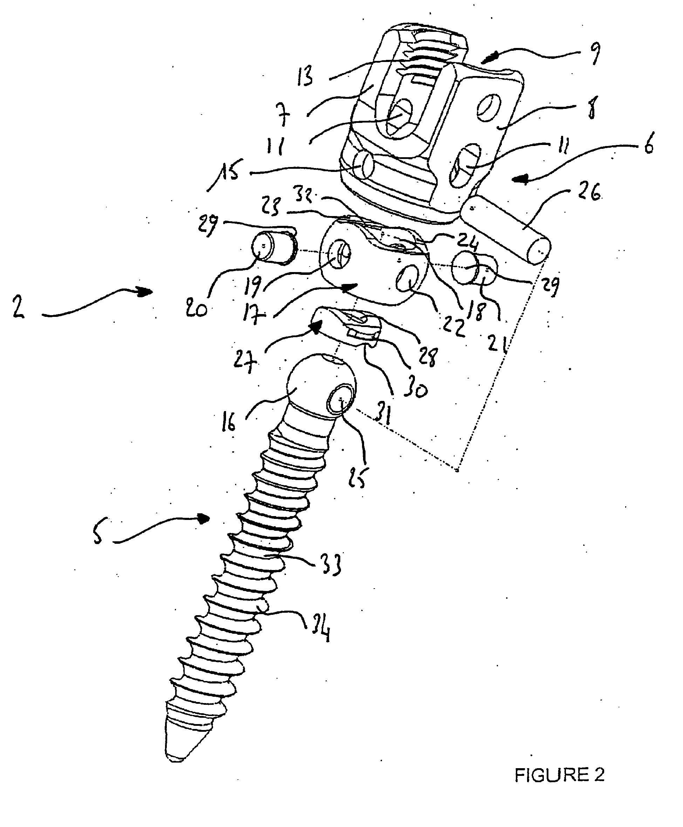

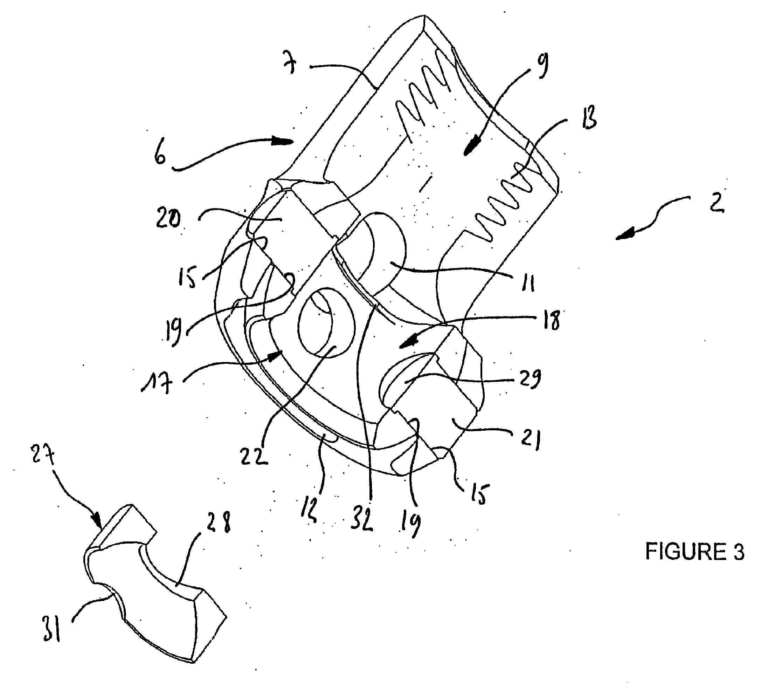

On a montré en figures 1 à 4 un dispositif d'ancrage vertébral 1 comportant des connecteurs 2 reliés entre eux par des tiges de liaison 3, tandis que chaque connecteur 2 est fixé dans chaque vertèbre 4 d'un segment rachidien à corriger par une vis d'ancrage 5.

Chaque connecteur 2 est constitué d'un élément de connexion 6 comportant des branches verticales supérieures 7 et 8 délimitant une ouverture 9 en forme de U, dont le fond 10 communique à l'opposé de l'ouverture 9 avec un logement 12 débouchant vers l'extérieur et dont le profil interne peut être en portion de sphère.

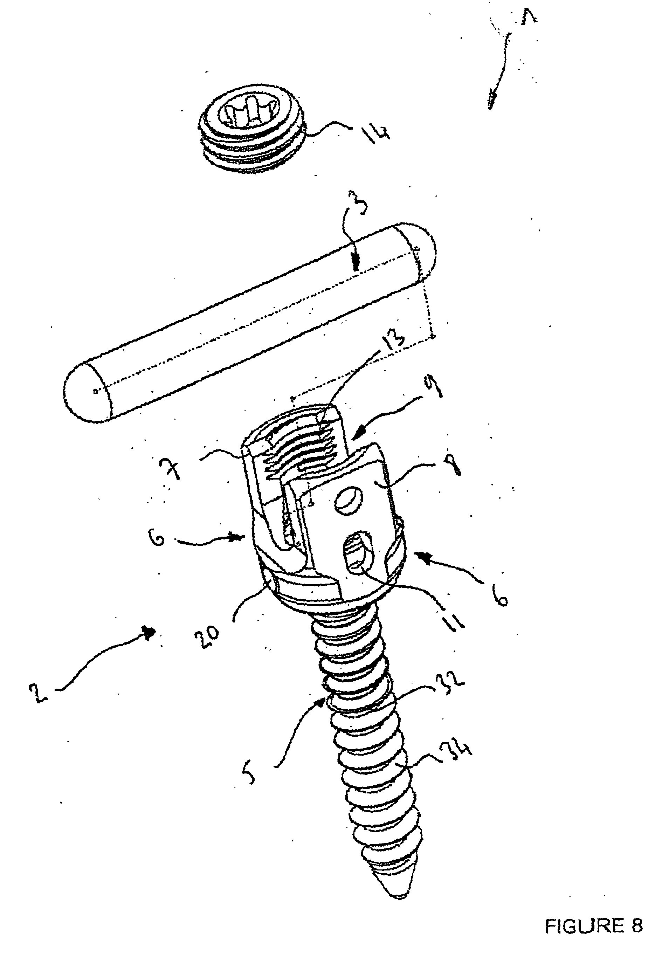

La partie interne des branches verticales supérieures 7 et 8 de l'élément de connexion 6 comporte au-dessus du fond 10 de l'ouverture 9 une section filetée 13 permettant le serrage d'une vis de pression 14 pour le blocage en translation et en rotation dans le fond du U de chaque connecteur 2 d'une tige de liaison 3 (figure 8).

L'élément de connexion 6 comprend en dessous de la section filetée 13 et à la base de chaque branche verticale 7, 8 une ouverture 11 débouchante, suivant une direction horizontale et perpendiculaire à celle de la tige de liaison 3, à l'intérieur dudit élément de connexion au niveau de la communication entre le fond 10 de l'ouverture 9 et le logement 12.

Chaque ouverture 11 disposée l'une en face de l'autre présente un profil vertical oblong de hauteur suffisante pour permettre lors de l'assemblage du connecteur 2 la mise en place d'un axe de rotation 26.

Le logement 12 de l'élément de connexion 6 comporte suivant une direction horizontale et perpendiculaire à celle des ouvertures oblongues 11 des alésages débouchants et coaxiaux 15.

Chaque connecteur 2 comporte une douille 17 présentant un profil externe complémentaire à celui interne du logement 12 ménagé dans la partie inférieure de l'élément de connexion 6.

La douille 17 est aménagée en son milieu et suivant un axe vertical d'un alésage interne 18 dans lequel est logé et immobilisé la vis d'ancrage 5 et plus particulièrement la tête 16 de cette dernière.

La douille 17 est percée d'une part suivant une première direction horizontale d'un premier alésage 19 débouchant à l'intérieur de l'alésage interne 18 et d'autre part suivant une seconde direction horizontale et perpendiculaire à la première d'un second alésage 22 débouchant également à l'intérieur de l'alésage interne 18.

La douille 17 comporte un bord périphérique supérieur 23 présentant au-dessus de chaque premier alésage débouchant 19 un bossage 24.

La douille 17 comporte au-dessus des seconds alésages 22 et niveau du bord supérieur 23 et à l'intérieur de l'alésage 18 des nervures 32.

Chaque connecteur 2 comporte une bague 27 traversée en son milieu et suivant une direction verticale par un trou 28.

La bague 27 comporte sur son pourtour externe des encoches 30 en dessous desquelles sont aménagées des échancrures en portion de cylindre 31.

Les éléments de connexion 6 de chaque connecteur 2 sont fixés dans chaque vertèbre 4 par la vis d'ancrage 5. Chaque vis d'ancrage 5 comporte une tête 16 se prolongeant par un corps cylindrique 33 présentant sur sa périphérie externe un filetage 34.

La tête 16 de la vis d'ancrage 5 présente un profil externe qui peut être de forme complémentaire ou non à celui de l'alésage interne 18 de la douille 17.

La tête 16 de la vis d'ancrage 5 est percée d'un alésage débouchant 25 destiné à coopérer avec l'axe de fixation 26 lors de sa mise en place à l'intérieur de l'élément de connexion 6 de chaque connecteur 2.

Ainsi le montage de chaque connecteur 2 du dispositif d'ancrage vertébral 1 autour de la vis d'ancrage 5 se comprend facilement de la description qui précède.

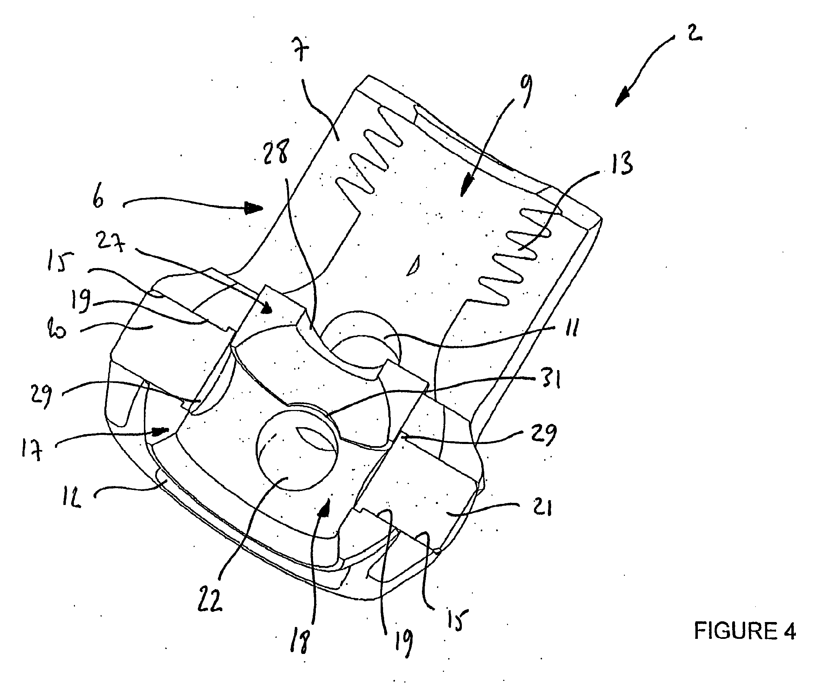

En premier lieu, la douille 17 est introduite à l'intérieur du logement 12 de l'élément de connexion 6 de manière à ce que chaque premier alésage 19 soit disposé en face de ceux 15 dudit logement (figure 4).

La douille 17 est immobilisée à l'intérieur du logement 12 par l'intermédiaire de deux axes de rotation 20, 21 qui sont respectivement introduits par l'intérieur de l'alésage 18 de la douille 17 pour venir coopérer avec les alésages 19 et 15 prévus à cet effet.

Les axes de rotation 20, 21 comportent une tête 29 dont le diamètre externe est supérieur à celui interne des alésages 19, 15 pour empêcher toute translation des ces derniers.

L'introduction des axes de rotation 20,21 dans les alésages horizontaux 19, 15 permet d'une part de retenir la douille 17 dans le logement 12 de l'élément de connexion 6 et d'autre part d'assurer une liberté de mouvement, c'est à dire une rotation ou un débattement angulaire autour desdits axes horizontaux 20, 21 entre ladite douille 17 et l'élément de connexion 6 (figure 4).

En second lieu, la bague 27 est introduite immobilisée à l'intérieur de l'alésage interne 18 de la douille 17 au moyen des encoches 30 qui viennent coopérer avec les nervures 32 prévues au niveau du bord supérieur 23 (figure 4).

La bague 27 est immobilisée à l'intérieur de la douille 17 permettant d'une part que les échancrures en portion de cylindre 31 soient disposées au-dessus des alésages horizontaux 22 aménagés dans ladite douille 17 et d'autre part que le pourtour externe de ladite bague opposé aux dites échancrures vienne en appui contre les axes de rotation 20, 21 afin d'empêcher ces derniers de se déplacer à l'intérieur des alésages 19 et 15.

La bague 27 est fixée dans la douille 17 de manière à prolonger cette dernière au-dessus de son bord supérieur 23 afin que ladite bague 27 vienne se positionner au niveau du fond 10 de l'ouverture 9 de l'élément de connexion 6.

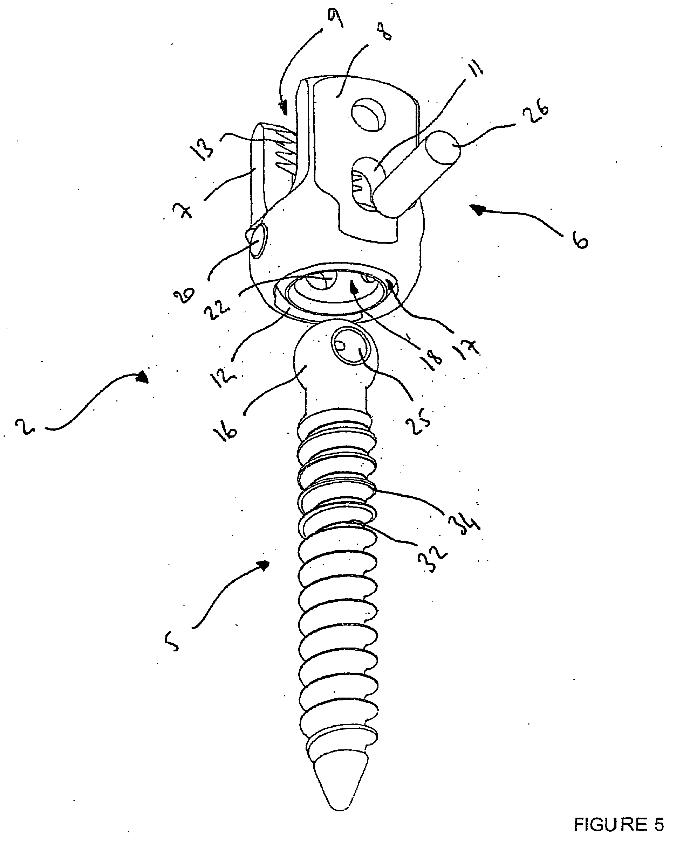

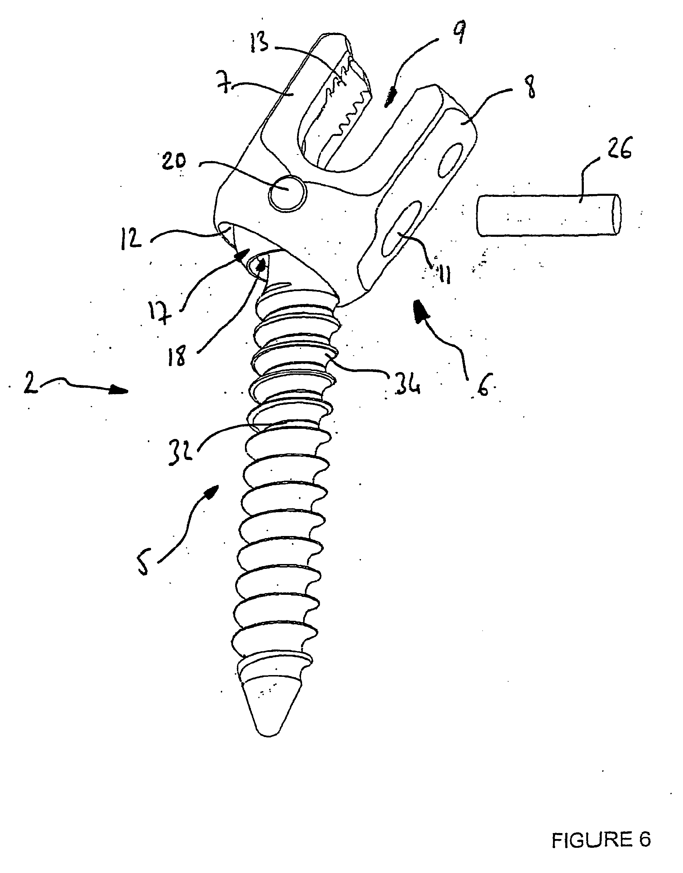

En troisième lieu, la tête 16 de la vis d'ancrage 5 est introduite à l'intérieur de l'alésage 18 de la douille 17 retenue à l'intérieur du logement 12 de l'élément de connexion 6. Pour cela, la douille 17 doit être positionnée de manière à ce que l'ouverture inférieure de l'alésage 18 soit dans l'alignement de celle du logement 12 de l'élément de connexion 6 (figures 5 et 6).

La tête 16 est introduite à l'intérieur de l'alésage 18 de la douille 17 afin que son alésage traversant 25 soit positionné en face et dans le prolongement de ceux 22 de ladite douille 17.

Lorsque la tête 16 présente un profil externe complémentaire à celui interne de l'alésage 18 de la douille 17, la mise en place de ladite tête ne peut s'effectuer que dans une seule position entraînant automatiquement l'alignement des alésages 25 de la tête 16 avec celui 22 de la douille 17.

Ensuite, l'élément de connexion 6 est incliné latéralement autour des axes de rotation 20, 21 tout en maintenant le corps cylindrique 33 de la vis d'ancrage 5 et la douille 17 dans une position verticale (figure 6).

Ce premier basculement latéral permet de positionner le premier alésage 22 de la douille 17 et une première extrémité de l'alésage 25 de la tête 16, avec l'une des ouvertures oblongues 11 ménagée dans l'élément de connexion 6.

Cette position permet l'introduction de l'axe de fixation 26, afin que ce dernier traverse l'ouverture oblongue 11 correspondante de l'élément de connexion 6 et vienne se loger totalement à l'intérieur des alésages 22 de la douille 17 et de l'alésage 25 de la tête 16 de la vis d'ancrage 5.

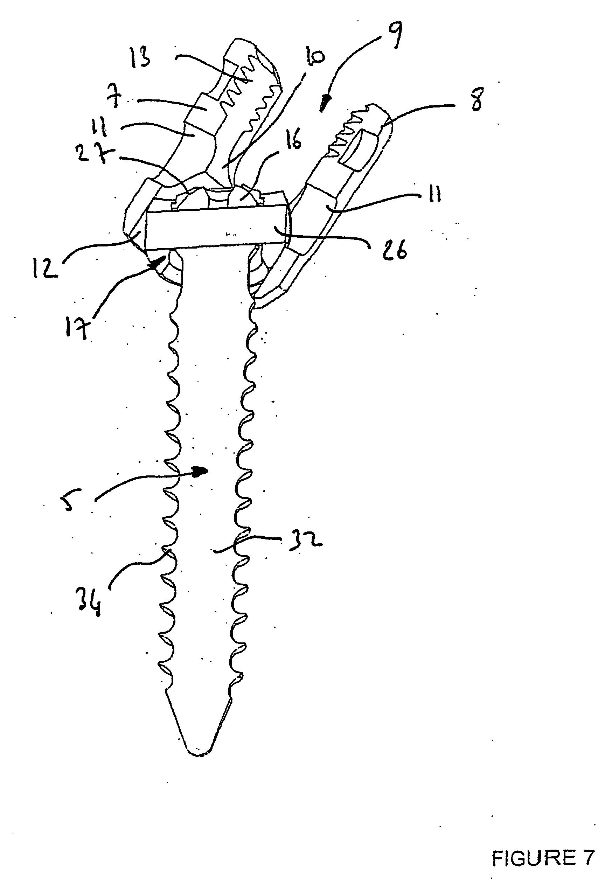

Ainsi, l'axe de fixation 26 est totalement introduit à l'intérieur des alésages 22 de la douille 17 et de l'alésage 25 de la tête 16 de la vis d'ancrage 5, permettant de retenir ladite vis d'ancrage 5 à l'intérieur de la douille 17 (figure 7).

On note que les dimensions de l'axe de fixation 26 sont inférieures à celles externes de la douille 17 pour que, une fois ce dernier totalement introduit dans les alésages 22 de la douille 17 et de l'alésage 25 de la tête 16 de la vis d'ancrage 5, la douille 17 et l'élément de connexion 6 puissent librement basculer autour des axes de rotation 20, 21.

On remarque que chaque connecteur 2 du dispositif d'ancrage vertébral 1 présente :
- un premier basculement latéral autour des axes de rotation 20, 21 reliant la douille 17 à l'élément de connexion 6,
- et un second basculement latéral autour de l'axe de retenue 26 reliant la tête 16 de la vis d'ancrage 5 à la douille 17, sachant que l'axe de retenue 26 est disposé dans une direction qui est horizontale et perpendiculaire à celle des axes horizontaux de rotation 20, 21.

On constate que la liaison de la vis d'ancrage 5 avec la douille 17 et de cette dernière avec l'élément de connexion 6 par l'intermédiaire des axes horizontaux 20, 21 et 26, permet d'assurer également l'entraînement en rotation autour de son axe longitudinal de la vis d'ancrage 5 pour la pénétration de son corps cylindrique 33 à l'intérieur du corps vertébral correspondant ou vertèbre 4.

L'entraînement en rotation de la vis d'ancrage 5 pour sa fixation dans la vertèbre 4 correspondante est assuré par un outil particulier et non représenté coopérant avec l'élément de connexion 6.

Lorsque la vis d'ancrage 5 de chaque connecteur 2 est fixée dans le corps vertébral 4, l'élément de connexion 6 est positionné dans la meilleure position angulaire par basculement autour des axes horizontaux 20, 21 et 26 afin de recevoir la tige de liaison 3 reliant chaque connecteur 2 du segment rachidien instrumenté de la colonne vertébrale.

La tige de liaison 3 est immobilisée à l'intérieur de chaque connecteur 2 en rotation autour de son axe longitudinal et en translation par l'intermédiaire de la vis de pression 14 qui est vissée entre les branches verticales supérieures 7 et 8 de l'élément de connexion 6 (figures 8 à10).

L'immobilisation de la tige de liaison 3 dans le fond de l'ouverture 9 de chaque élément de connexion 6 par la vis de pression 14 permet également de bloquer simultanément l'élément de connexion 6 dans une position angulaire adéquate autour de la tête 16 de la vis d'ancrage 5.

En effet, la bague 27 permet par l'intermédiaire de son alésage central 28 à la tête 16 de la vis d'ancrage 5 de venir au niveau du fond 10 de l'ouverture 9 recevant la tige de liaison 3.

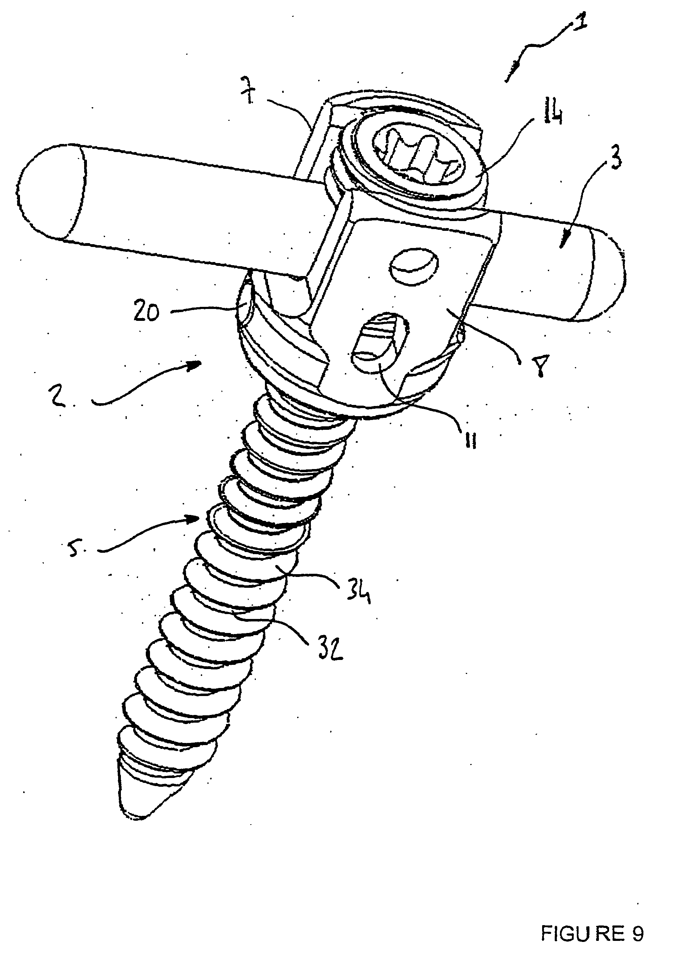

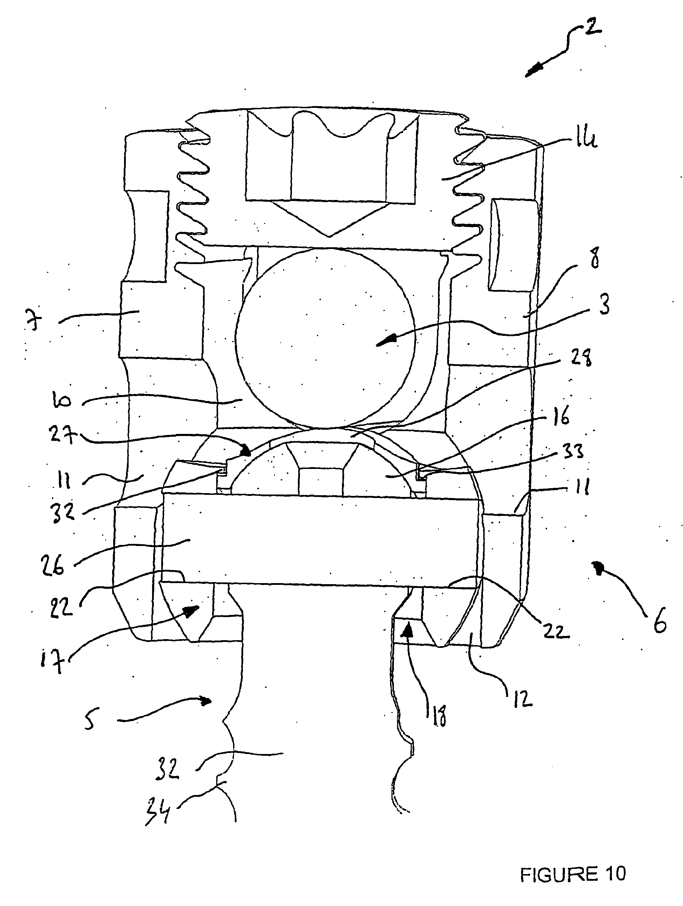

La fixation de la tige de liaison 3 entre les branches verticales 7, 8 et contre le fond 10 de l'ouverture 9 au moyen de la vis de pression 14 permet à cette dernière de venir en appui contre la tête 16 de la vis d'ancrage 5 et de bloquer l'élément de connexion 6 (figure 9 et 10).

Il doit d'ailleurs être entendu que la description qui précède n'a été donnée qu'a titre d'exemple et qu'elle ne limite nullement le domaine de l'invention dont on ne sortirait pas en remplaçant les détails d'exécution décrits par tous autres équivalents.

## Revendications

1. Dispositif d'ancrage vertébral comprenant des connecteurs (2) reliés entre eux par des tiges de liaison (3) et fixés sur des vis d'ancrage osseux (5) implantés dans les vertèbres (4) d'une colonne vertébrale, chaque connecteur (2) étant constitué d'un élément de connexion (6) comportant d'une part des moyens de réception et de fixation permettant l'immobilisation en translation et en rotation de la tige de liaison (3) et d'autre part à l'opposé des moyens de réception et de fixation de la tige de liaison (3), un logement (12) coopérant avec un dispositif de liaison constitué d'une douille (17) permettant l'assemblage de la vis d'ancrage (5), **caractérisé en ce que** la douille (17) assure avant l'immobilisation de la tige de liaison (3), dans l'élément de connexion (6),
• d'une part des basculements latéraux et indépendants de l'élément de connexion (6) et de la vis d'ancrage (5) l'un par rapport à l'autre par l'intermédiaire de premiers moyens de rotation constitués de premiers alésages (15, 19) ménagés respectivement dans le logement (12) de l'élément de connexion (6) et la douille (17) du dispositif de liaison permettant à ladite douille de pouvoir basculer latéralement autour d'axes de rotation (20, 21) coopérant avec lesdits premiers alésage (15, 19) et de seconds moyens de rotation constitués de second alésages (22, 25) ménagés respectivement dans la douille (17) et la tête (16) de la vis d'ancrage (5) permettant à cette dernière de pouvoir basculer latéralement autour d'un autre axe de rotation (26),
• Et d'autre part l'entraînement en rotation de la vis d'ancrage (5) pour sa fixation dans le corps de la vertèbre (4).

2. Dispositif d'ancrage vertébral suivant la revendication 1, **caractérisé en ce que** la douille (17) est percée en son milieu et suivant une direction verticale d'un alésage débouchant (18) destiné à recevoir la tête (16) de la vis d'ancrage (5).

3. Dispositif d'ancrage vertébral suivant la revendication 1, **caractérisé en ce que** la douille (17) est percée suivant une première direction horizontale d'un premier alésage (19) débouchant à l'intérieur de l'alésage interne (18) et permettant la mise en place des axes de rotation (20, 21) afin que ces derniers débouchent dans les alésages (15) aménagés dans le logement (12) de l'élément de connexion (6) afin d'assurer le premier basculement latéral.

4. Dispositif d'ancrage vertébral suivant la revendication 1, **caractérisé en ce que** la douille (17) est percée suivant une seconde direction horizontale et perpendiculaire à celle du premier alésage (19) d'un second alésage (22) débouchant également à l'intérieur de l'alésage interne (18) et permettant la mise en place d'un axe de fixation (26) traversant un alésage (25) ménagé dans la tête (16) de la vis d'ancrage (5) afin d'assurer le second basculement latéral.

5. Dispositif d'ancrage vertébral suivant la revendication 1, **caractérisé en ce que** la douille (17) comporte un bord périphérique supérieur (23) présentant d'une part au-dessus de chaque premier alésage débouchant (19) un bossage (24) et d'autre part au-dessus de chaque second alésage (22) et à l'intérieur de l'alésage interne (18) des nervures (32).

6. Dispositif d'ancrage vertébral suivant la revendication 5, **caractérisé en ce que** la douille (17) est reliée à une bague (27) comportant en son milieu et suivant une direction verticale un trou (28) et sur son pourtour externe des encoches (30) coopérant avec les nervures (32) de ladite douille.

7. Dispositif d'ancrage vertébral suivant la revendication 1, **caractérisé en ce que** l'élément de connexion (6) comporte des moyens de réception et de fixation de la tige de liaison (3) qui sont constitués par des branches verticales supérieures (7, 8) délimitant une ouverture (9) en forme de U pour la réception de ladite tige de liaison, lesdites branches verticales supérieures (7, 8) comprenant au-dessus du fond (10) de l'ouverture (9) une section filetée (13) permettant le serrage d'une vis de pression (14) pour le blocage en translation et en rotation de la tige de liaison (3).

8. Dispositif d'ancrage vertébral suivant la revendication 7, **caractérisé en ce que** l'élément de connexion (6) comprend en dessous de la section filetée (13) et à la base de chaque branche verticale (7, 8) une ouverture (11) débouchant à l'intérieur dudit élément de connexion au niveau de la communication entre le fond (10) de l'ouverture (9) et le logement (12) suivant une direction horizontale et perpendiculaire à celle de la tige de liaison (3).

9. Dispositif d'ancrage vertébral suivant la revendication 8, **caractérisé en ce que** chaque ouverture (11) disposée l'une en face de l'autre présente un profil vertical oblong.

10. Dispositif d'ancrage vertébral suivant les revendications 3 et 8, **caractérisé en ce que** les alésages débouchants (15) du logement (12) de l'élément de connexion (6) sont disposés suivant une direction qui est horizontale et perpendiculaire à celle des ouvertures oblongues (11).

## Claims

1. Vertebral anchoring device comprising connectors (2) that are linked to one another by connecting rods (3) and are fixed on bone-anchoring screws (5) implanted in the vertebrae (4) of a vertebral column, each connector (2) being composed of a connection element (6) that comprises, on the one hand, receiving and fixing means to permit immobilization of the connecting rod (3) in translation and in rotation, and on the other hand, opposite the means for receiving and fixing the connecting rod (3), a seat (12) that cooperates with a connecting device composed of a bushing (17) permitting assembly of the anchoring screw (5), **characterized in that**, before the immobilization of the connecting rod (3) in the connection element (6), the bushing (17) permits
• on the one hand, lateral and independent tilting movements of the connection element (6) and of the anchoring screw (5) relative to each other by way of first means of rotation composed of first bores (15, 19), which are formed respectively in the seat (12) of the connection element (6) and the bushing (17) of the connecting device and allow said bushing to tilt laterally about rotation pins (20, 21) cooperating with said first bores (15, 19), and second means of rotation composed of second bores (22, 25), which are formed respectively in the bushing (17) and the head (16) of the anchoring screw (5) and allow the latter to tilt laterally about another rotation pin (26),
• and, on the other hand, rotation of the anchoring screw (5) for fixing it in the body of the vertebra (4).

2. Vertebral anchoring device according to Claim 1, **characterized in that** the bushing (17) has at its center, and extending in a vertical direction, a through-bore (18) designed to receive the head (16) of the anchoring screw (5).

3. Vertebral anchoring device according to Claim 1, **characterized in that** the bushing (17) has, extending in a first horizontal direction, a first bore (19) that opens into the inner bore (18) and permits placement of the rotation pins (20, 21), such that the latter open into the bores (15) formed in the seat (12) of the connection element (6), so as to permit the first lateral tilting movement.

4. Vertebral anchoring device according to Claim 1, **characterized in that** the bushing (17) has, extending in a second horizontal direction perpendicular to that of the first bore (19), a second bore (22) that also opens into the inner bore (18) and permits placement of a fixation pin (26) that passes through a bore (25) formed in the head (16) of the anchoring screw (5), so as to permit the second lateral tilting movement.

5. Vertebral anchoring device according to Claim 1, **characterized in that** the bushing (17) comprises an upper peripheral edge (23) which has, on the one hand, a hump (24) arranged above each first through-bore (19) and, on the other hand, ribs (32) arranged above each second bore (22) and inside the inner bore (18).

6. Vertebral anchoring device according to Claim 5, **characterized in that** the bushing (17) is joined to a ring (27) which, at its center, has a hole (28) extending in a vertical direction, and, on its outer circumference, has recesses (30) cooperating with the ribs (32) of said bushing.

7. Vertebral anchoring device according to Claim 1, **characterized in that** the connection element (6) comprises means for receiving and fixing the connecting rod (3), said means being composed of upper vertical branches (7, 8) that delimit a U-shaped opening (9) for receiving said connecting rod, said upper vertical branches (7, 8) comprising, above the bottom (10) of the opening (9), a threaded section (13) that allows a press-screw (14) to be tightened in order to block the connecting rod (3) in translation and in rotation.

8. Vertebral anchoring device according to Claim 7, **characterized in that** the connection element (6) comprises, below the threaded section (13) and at the base of each vertical branch (7, 8), an opening (11) that opens into the inside of said connection element, in the area of the communication between the bottom (10) of the opening (9) and the seat (12), in a horizontal direction perpendicular to that of the connecting rod (3).

9. Vertebral anchoring device according to Claim 8, **characterized in that** each of the openings (11) arranged opposite each other has an oblong vertical profile.

10. Vertebral anchoring device according to Claims 3 and 8, **characterized in that** the through-bores (15) of the seat (12) of the connection element (6) are arranged in a direction that is horizontal and perpendicular to that of the oblong openings (11).

## Patentansprüche

1. Vorrichtung zur Wirbelfixation, umfassend Verbindungsstücke (2), die miteinander durch Verbindungsstangen (3) verbunden und an Knochenfixationsschrauben (5) befestigt sind, die in den Wirbeln (4) einer Wirbelsäule eingesetzt sind, wobei jedes Verbindungsstück (2) von einem Anschlusselement (6) gebildet ist, umfassend einerseits Aufnahme- und Befestigungsmittel, die die Feststellung der Verbindungsstange (3) in Translation und in Drehung ermöglichen, und andererseits gegenüber den Aufnahme- und Befestigungsmitteln der Verbindungsstange (3) eine Lagerung (12), die mit einer Verbindungsvorrichtung zusammenwirkt, die von einer Büchse (17) gebildet ist, die die Befestigung der Fixationsschraube (5) ermöglicht, **dadurch gekennzeichnet, dass** die Büchse (17) vor der Feststellung der Verbindungsstange (3) in dem Anschlusselement (6) Folgendes gewährleistet:
- einerseits seitliche und unabhängige Kippbewegungen des Anschlusselements (6) und der Fixationsschraube (5) zueinander mit Hilfe von ersten Rotationsmitteln, die von ersten Bohrungen (15, 19) gebildet sind, die in der Lagerung (12) des Anschlusselements (6) bzw. der Büchse (17) der Verbindungsvorrichtung vorgesehen sind und es der Büchse ermöglichen, seitlich um Rotationsachsen (20, 21), die mit den ersten Bohrungen (15, 19) zusammenwirken, schwenken zu können, und von zweiten Rotationsmitteln, die von zweiten Bohrungen (22, 25) gebildet sind, die in der Büchse (17) bzw. dem Kopf (16) der Fixationsschraube (5) vorgesehen sind und es dieser letztgenannten ermöglichen, seitlich um eine Rotationsachse (26) zu schwenken,
- und andererseits den Rotationsantrieb der Fixationsschraube (5) für ihre Befestigung im Wirbelkörper (4).

2. Vorrichtung zur Wirbelfixation nach Anspruch 1, **dadurch gekennzeichnet, dass** die Büchse (17) in ihrer Mitte und entlang einer Vertikalrichtung eine durchgehende Bohrung (18) aufweist, die dazu bestimmt ist, den Kopf (16) der Fixationsschraube (5) aufzunehmen.

3. Vorrichtung zur Wirbelfixation nach Anspruch 1, **dadurch gekennzeichnet, dass** die Büchse (17) entlang einer ersten Horizontalrichtung eine erste durchgehende Bohrung (19) im Inneren der Innenbohrung (18) aufweist, die die Anbringung der Rotationsachsen (20, 21) ermöglicht, damit diese letztgenannten in die Bohrungen (15) münden, die in der Lagerung (12) des Anschlusselements (6) vorgesehen sind, um das erste seitliche Schwenken zu gewährleisten.

4. Vorrichtung zur Wirbelfixation nach Anspruch 1, **dadurch gekennzeichnet, dass** die Büchse (17) entlang einer zweiten Horizontalrichtung, die zu jener der ersten Bohrung (19) senkrecht ist, eine zweite durchgehende Bohrung (22) ebenfalls im Inneren der Innenbohrung (18) aufweist, die die Anbringung einer Fixationsachse (26) ermöglicht, die durch eine Bohrung (25) hindurchgeht, die in dem Kopf (16) der Fixationsschraube (5) vorgesehen ist, um das zweite seitliche Schwenken zu gewährleisten.

5. Vorrichtung zur Wirbelfixation nach Anspruch 1, **dadurch gekennzeichnet, dass** die Büchse (17) einen oberen Umfangsrand (23) umfasst, der einerseits über jeder ersten durchgehenden Bohrung (19) einen Wulst (24) und andererseits über jeder zweiten Bohrung (22) und im Inneren der Innenbohrung (18) Rippen (32) aufweist.

6. Vorrichtung zur Wirbelfixation nach Anspruch 5, **dadurch gekennzeichnet, dass** die Büchse (17) mit einem Ring (27) verbunden ist, der in seiner Mitte und entlang einer Vertikalrichtung ein Loch (28) und auf seinem äußeren Umfang Kerben (30) aufweist, die mit den Rippen (32) der Büchse zusammenwirken.

7. Vorrichtung zur Wirbelfixation nach Anspruch 1, **dadurch gekennzeichnet, dass** das Anschlusselement (6) Mittel zur Aufnahme und Befestigung der Verbindungsstange (3) umfasst, die von oberen vertikalen Abschnitten (7, 8) gebildet sind, die eine U-förmige Öffnung (9) für die Aufnahme der Verbindungsstange begrenzen, wobei die oberen vertikalen Abschnitte (7, 8) über dem Boden (10) der Öffnung (9) einen Gewindeteil (13) umfassen, der die Befestigung einer Druckschraube (14) zur Feststellung der Verbindungsstange (3) in Translation und Rotation ermöglicht.

8. Vorrichtung zur Wirbelfixation nach Anspruch 7, **dadurch gekennzeichnet, dass** das Anschlusselement (6) unter dem Gewindeteil (13) und an der Basis jedes vertikalen Abschnitts (7, 8) eine durchgehende Öffnung (11) im Inneren des Anschlusselements im Bereich der Verbindung zwischen dem Boden (10) der Öffnung (9) und der Lagerung (12) entlang einer horizontalen und zu jener der Verbindungsstange (3) senkrechten Richtung umfasst.

9. Vorrichtung zur Wirbelfixation nach Anspruch 8, **dadurch gekennzeichnet, dass** jede Öffnung (11), die gegenüber der anderen angeordnet ist, ein längliches vertikales Profil aufweist.

10. Vorrichtung zur Wirbelfixation nach den Ansprüchen 3 und 8, **dadurch gekennzeichnet, dass** die durchgehenden Bohrungen (15) der Lagerung (12) des Anschlusselements (6) entlang einer Richtung angeordnet sind, die horizontal und zu jener der länglichen Öffnungen (11) senkrecht ist.
